# EUROPÄISCHE PATENTANMELDUNG

(11) **EP 4 652 955 A1**
(43) Veröffentlichungstag der Anmeldung: **26.11.2025**
(21) Anmeldenummer: 25169168.9
(22) Anmeldetag: 08.04.2025
(51) Int. Cl.: A61B 34/30, A61B 34/37, A61B 17/00, A61B 46/00

(54) **SCHNITTSTELLE ZUR VERBINDUNG EINER ANTRIEBSEINHEIT MIT EINEM MEDIZINISCHEN INSTRUMENT**

(30) Priorität: 24.05.2024 DE 102024114716
(71) Anmelder: Karl Storz SE & Co. KG, 78532 Tuttlingen (DE)
(72) Erfinder: HOFFMANN, Martin, 13187 Berlin (DE); RODRIGUEZ, Jerick, 81245 München (DE)

(57) **Zusammenfassung**

Die Erfindung betrifft eine Schnittstelle (10) zur Verbindung einer Antriebseinheit (12) mit einem medizinischen Instrument (14). Die Schnittstelle umfasst:
- wenigstens eine antriebsseitige Kopplungsvorrichtung (16), und
- wenigstens eine zu der antriebsseitigen Kopplungsvorrichtung (16) komplementäre instrumentenseitige Kopplungsvorrichtung (20).

Die antriebsseitige und die instrumentenseitige Kopplungsvorrichtung (16, 20) sind unter Zwischenschaltung einer durchgängigen medizinischen Barriere (24) mechanisch miteinander koppelbar. Zumindest die antriebsseitige Kopplungsvorrichtung (16) umfasst wenigstens eine Antriebsanordnung (26), welche dazu eingerichtet ist, zumindest in einem Abschnitt der medizinischen Barriere (24) eine wellenförmige Bewegung der medizinischen Barriere (24) zu erzeugen.

## Beschreibung

Die vorliegende Erfindung betrifft eine Schnittstelle zur Verbindung einer Antriebseinheit mit einem medizinischen Instrument, insbesondere als Teil eines medizinischen Robotersystems.

Medizinische Robotersysteme, insbesondere in Form von herkömmlichen medizinischen Master-Slave Robotersystemen, die beispielsweise für die mechanische Führung von medizinischen und/oder chirurgischen Instrumenten sowie die Steuerung derer Endeffektoren eingesetzt werden, weisen in der Regel eine lösbare Verbindung an einer Schnittstelle zwischen motorischen Antrieben auf der Roboterseite und den chirurgischen Instrumenten auf. Dabei werden die Antriebseinheit und das chirurgische Instrument meist über eine Getriebeanordnung kraftschlüssig miteinander gekoppelt.

Um Anforderungen an eine sterile Behandlungsumgebung erreichen zu können, ist im Bereich der Schnittstelle eine medizinische Barriere üblicherweise in Form einer Kunststofffolie vorgesehen, welche einen antriebsseitigen, in der Regel unsterilen, Teil der Schnittstelle von einem instrumentenseitigen sterilen Teil der Schnittstelle trennt bzw. abdeckt. Zur sterilen Kopplung einer Schnittstelle bzw. zur Übertragung zumindest einer Antriebskraft und/oder zumindest eines Antriebsdrehmoments zwischen den Komponenten der Schnittstelle, weisen bekannte medizinische Barrieren komplex geformte Folien oder speziell eingebettete Adapter in der Folie auf, über welche die Antriebskraft und/oder das Antriebsdrehmoment übertragbar sind. Derartige Schnittstellen bzw. dafür entwickelte medizinische Barrieren sind beispielsweise in den Dokumenten WO 2007/126443 A2 und US 2023/0390016 A1 beschrieben. Die komplex geformten Folien und/oder in die Folie integrierte Adapter verteuern die medizinische Barriere. Ferner müssen die Barrieren zudem punktgenau platziert werden, wodurch sich ein Arbeitsaufwand im Vorfeld eines medizinischen und/oder chirurgischen Eingriffs erhöht.

Ausgehend vom Stand der Technik liegt der Erfindung insbesondere aber nicht beschränkt darauf die Aufgabe zugrunde, eine Schnittstelle zur Verbindung einer Antriebseinheit mit einem medizinischen Instrument vorteilhaft weiterzuentwickeln, insbesondere hinsichtlich von Kosten und/oder einer Installation bzw. Deinstallation. Ferner ist es insbesondere eine Aufgabe der vorliegenden Erfindung, eine zuverlässige und funktionsfähige Schnittstelle mit optimierten Eigenschaften bereitzustellen, sodass insbesondere vor einem medizinischen Eingriff und/oder während eines medizinischen Eingriffs an einem Patienten stets ein steriles Arbeitsfeld gewährleistet bzw. sichergestellt werden kann.

Diese Aufgabe wird erfindungsgemäß durch die Merkmale der unabhängigen Ansprüche gelöst. Weiterbildungen der Erfindung sind den abhängigen Ansprüchen zu entnehmen.

Die Erfindung betrifft eine Schnittstelle zur Verbindung einer Antriebseinheit mit einem medizinischen Instrument umfassend:
- wenigstens eine antriebsseitige Kopplungsvorrichtung, und
- wenigstens eine zu der antriebsseitigen Kopplungsvorrichtung komplementäre instrumentenseitige Kopplungsvorrichtung,

wobei die antriebsseitige und die instrumentenseitige Kopplungsvorrichtung unter Zwischenschaltung einer durchgängigen medizinischen Barriere mechanisch miteinander koppelbar sind, und
wobei zumindest die antriebsseitige Kopplungsvorrichtung wenigstens eine Antriebsanordnung umfasst, welche dazu eingerichtet ist, zumindest in einem Abschnitt der medizinischen Barriere eine wellenförmige Bewegung der medizinischen Barriere zu erzeugen.

Die Erfindung betrifft ferner ein medizinisches Robotersystem, welches eine Schnittstelle der vorangehend beschriebenen Art umfasst.

Durch eine derartige Ausgestaltung kann eine vorteilhaft weiterentwickelte Schnittstelle sowie ein vorteilhaft weiterentwickeltes medizinisches Robotersystem bereitgestellt werden. Insbesondere kann eine Installation bzw. Deinstallation medizinischer Instrumente vereinfacht und es können Kosten sowie Stillstandszeiten reduziert werden, da eine durchgängige medizinische Barriere zum Einsatz kommen kann, die insbesondere "einfach geformt ist und/oder auf speziell eingebettete Adapter zur Übertragung einer Antriebskraft und/oder eines Antriebsdrehmoments verzichten kann. Hierdurch kann eine zeitaufwändige präzise Platzierung der medizinischen Barriere entfallen. Zudem kann ein Kostenaufwand für jeden medizinischen Einsatz reduziert werden, da eine nur einmalig verwendbare medizinische Barriere möglichst kostengünstig gehalten werden kann. Insbesondere kann eine medizinische Standardbarriere zum Einsatz kommen. Ferner kann durch die erfindungsgemäßen Merkmale eine besonders zuverlässige und funktionsfähige Schnittstelle mit optimierten Eigenschaften bereitgestellt werden, sodass insbesondere vor einem medizinischen Eingriff und/oder während eines medizinischen Eingriffs an einem Patienten stets ein steriles Arbeitsfeld gewährleistet und eine zuverlässige mechanische Kopplung sichergestellt ist.

Die Schnittstelle kann Teil eines medizinischen Robotersystems sein. Das Robotersystem kann zumindest einen Roboterarm, eine Bedienkonsole, einen Elektronikschrank, insbesondere ein Elektronik-Rack, beispielsweise zur Aufnahme zusätzlicher Geräte wie eines HF-Generators, eine Anzeigeeinheit, eine Patientenliege, eine Aufbewahrungseinheit für verschiedene medizinische Instrumente zur Verwendung mit der Schnittstelle und/oder weitere, dem Fachmann als sinnvoll erscheinende Einheiten aufweisen. Die durchgängige medizinische Barriere kann ebenfalls Teil des medizinischen Robotersystems sein. **In** einem Betriebszustand kann die Schnittstelle durch den Roboterarm getragen und durch diesen bewegbar sein.

Bei dem medizinischen Instrument kann es sich um einen beliebigen, dem Fachmann als sinnvoll erscheinenden Effektor, insbesondere in Form eines medizinischen und/oder chirurgischen Instruments handeln, welches bevorzugt durch den Roboterarm getragen und durch diesen bewegbar sein kann. Das medizinische Instrument kann mit der instrumentenseitigen Kopplungsvorrichtung fest oder lösbar verbunden sein oder Teil der instrumentenseitigen Kopplungsvorrichtung sein. Das medizinische Instrument kann beispielsweise eine laparoskopische Einheit, ein Endoskop, ein Mikroskop, ein Exoskop, ein Skalpell, einen Bohrer, einen Schaber, eine Klammer, eine Zange, eine Schere, eine Spritze, einen Katheter und/oder weitere, dem Fachmann als sinnvoll erscheinende Einheiten aufweisen, welche mittels der Antriebseinheit über die Schnittstelle antreibbar und/oder ansteuerbar sein können.

In dem gekoppelten Zustand der Schnittstelle kontaktiert die antriebsseitige Kopplungsvorrichtung bzw. eine antriebsseitige Kopplungsfläche der antriebsseitigen Kopplungsvorrichtung eine erste Seite der medizinischen Barriere und die instrumentenseitige Kopplungsvorrichtung bzw. eine instrumentenseitige Kopplungsfläche der instrumentenseitigen Kopplungsvorrichtung eine der ersten Seite gegenüberliegende zweite Seite der medizinischen Barriere. Die Kopplungsvorrichtungen bzw. die Kopplungsflächen können dabei bevorzugt zueinander komplementär ausgestaltet sein, insbesondere um eine formschlüssige Aufnahme und/oder ein formschlüssiges Einklemmen der medizinischen Barriere zwischen den Verbindungselementen zu ermöglichen. Die Kopplungsflächen können jeweils eine Gesamtfläche von zumindest 50 mm², insbesondere von zumindest 100 mm² und beispielsweise von zumindest 250 mm² aufweisen.

Bei einer "Kopplungsvorrichtung" kann es sich um eine mechanische Einrichtung handeln, die dazu eingerichtet ist, zwei oder mehrere Bauteilkomponenten miteinander zu verbinden und/oder mit ein oder mehreren Bauteilkomponenten gekoppelt zu werden. Die Kopplungsvorrichtung kann dazu eingerichtet sein, eine feste Verbindung zwischen den Bauteilkomponenten herzustellen, sodass diese miteinander interagieren können, sei es mechanisch, elektrisch, hydraulisch und/oder auf eine andere Weise. Die Kopplungsvorrichtungen können mit ihren Kopplungsflächen zusammengesetzt einen um eine Drehsymmetrieachse zumindest teilweise rotationssymmetrischen, insbesondere zumindest teilweise kreiszylindrischen und/oder wenigstens teilweise kegelstumpfförmigen, Körper ausbilden. Die Kopplungsvorrichtungen können dabei zumindest teilweise quer zur Drehsymmetrieachse zusammengesetzt sein. In manchen Ausgestaltungen kann die antriebsseitige Kopplungsvorrichtung größer ausgestaltet sein als die instrumentenseitige Kopplungsvorrichtung. Die antriebsseitige Kopplungsvorrichtung kann einen Aufnahmeraum zur Aufnahme der instrumentenseitigen Kopplungsvorrichtung aufweisen.

Unter "eingerichtet" soll speziell programmiert, vorgesehen, ausgelegt, ausgebildet und/oder ausgestattet verstanden werden. Darunter, dass ein Objekt zu einer bestimmten Funktion eingerichtet ist, soll ferner verstanden werden, dass das Objekt diese bestimmte Funktion in zumindest einem Anwendungs- oder Betriebszustand erfüllt oder ausführt.

Die durchgängige medizinische Barriere kann frei von Öffnungen, Einlegern und/oder speziellen eingebetteten Adaptern zur Übertragung einer Antriebskraft und/oder eines Antriebsmoments sein. Die durchgängige medizinische Barriere kann über Ihre gesamte Flächenerstreckung eine Dicke von höchstens 1 mm, insbesondere von maximal 0,5 mm und beispielsweise von höchstens 0,1 mm aufweisen. Die durchgängige medizinische Barriere ist bevorzugt flexibel ausgebildet. Besonders bevorzugt ist die durchgängige medizinische Barriere als ein entsprechend bearbeitetes und/oder beschichtetes Gewebe und/oder vorzugsweise als eine Folie, insbesondere als eine Kunststofffolie, ausgebildet.

Dass die antriebsseitige und die instrumentenseitige Kopplungsvorrichtung mechanisch miteinander koppelbar sind, kann so verstanden werden, dass die antriebsseitige und die instrumentenseitige Kopplungsvorrichtung dazu eingerichtet sind, verliersicher und/oder funktional miteinander verbunden zu werden, um eine bestimmte Funktion zu ermöglichen. Durch die mechanische Kopplung und/oder durch die Verbindung können Kräfte und/oder Bewegungen zwischen den beiden Kopplungsvorrichtungen direktional oder bidirektional übertragen werden, was es ermöglicht, dass die beiden Kopplungsvorrichtungen zusammenarbeiten und/oder miteinander interagieren.

Die antriebsseitige Kopplungsvorrichtung und/oder die instrumentenseitige Kopplungsvorrichtung können ein Gehäuse umfassen. Das Gehäuse kann insbesondere dazu eingerichtet sein, Komponenten, die zumindest abschnittsweise von dem Gehäuse umgeben werden, gegen äußere Einflüsse wie Staub, Feuchtigkeit und/oder mechanische Beschädigungen zu schützen. Das Gehäuse kann aus verschiedenen Materialien wie Kunststoff, Metall oder Gummi bestehen. Das Gehäuse kann so konfiguriert sein, dass es einfach mit wenigstens einem anderen Gehäuse oder System verliersicher verbunden werden kann.

Die antriebsseitige Kopplungsvorrichtung umfasst wenigstens eine Antriebsanordnung. Die wenigstens eine Antriebsanordnung ist dazu eingerichtet, zumindest in einem Abschnitt der medizinischen Barriere eine wellenförmige Bewegung der medizinischen Barriere zu erzeugen. Die wenigstens eine Antriebsanordnung der antriebsseitigen Kopplungsvorrichtung ist insbesondere dazu eingerichtet in einem unter Zwischenschaltung der medizinischen Barriere mit der instrumentenseitigen Kopplungsvorrichtung mechanisch gekoppelten Zustand eine wellenförmige Bewegung der medizinischen Barriere zu erzeugen.

Unter einer "wellenförmigen Bewegung" kann eine in vorzugsweise regelmäßigen Abständen erfolgende Aufwärts- und Abwärtsbewegung bzw. eine lokale Bewegung von Teilbereichen der medizinischen Barriere senkrecht zu der Erstreckungsrichtung der medizinischen Barriere und/oder der Kopplungsflächen der Schnittstelle verstanden werden. Die charakteristischen Eigenschaften der durch die Antriebsanordnung erzeugten Wellenform der Barriere können beispielsweise durch die Amplitude und/oder die Wellenlänge einer auf der medizinischen Barriere erzeugten Welle beschrieben werden.

Die wellenförmige Bewegung der medizinischen Barriere kann periodisch sein und entlang einer Bezugsachse erfolgen, die zwischen und/oder parallel zu den Kopplungsflächen der Kopplungsvorrichtungen verläuft.

Gemäß einer Weiterbildung kann die wellenförmige Bewegung der medizinischen Barriere entlang wenigstens einer Ausbreitungsrichtung erfolgen. In anderen Worten kann sich die wellenförmige Bewegung, im Gegensatz zu einer stehenden Welle, entlang einer Ausbreitungsrichtung ausbreiten. Je nach Anforderung kann sich die wellenförmige Bewegung zusätzlich oder alternativ auch in einer der wenigstens einen Ausbreitungsrichtung entgegengesetzten Ausbreitungsrichtung erfolgen. Die wellenförmige Bewegung ist bevorzugt von einer stehenden Welle verschieden. Durch die wellenförmige Bewegung kann die medizinische Barriere gleichmäßig und schonend belastet werden. Da sich bei der sich wellenförmigen Bewegung der medizinischen Barriere die Beanspruchung des Materials der medizinischen Barriere ständig verlagert, kann das Risiko für Ermüdungserscheinungen in bestimmten Bereichen des Materials reduziert werden.

In einigen Ausführungsformen kann die Antriebsanordnung wenigstens zwei, insbesondere wenigstens 5, vorzugsweise wenigstens 10 und besonders bevorzugt wenigstens 20 Antriebselemente zu Erzeugung der wellenförmigen Bewegung umfassen. Eine hohe Anzahl an Antriebselementen einer Antriebsanordnung kann die Flexibilität und Beweglichkeit der Antriebsanordnung verbessern. Ferner kann eine hohe Anzahl an Antriebselementen dazu beitragen, dass Kräfte und/oder Bewegungen gleichmäßiger und/oder präziser übertragen werden können. Durch eine redundante Anordnung der Antriebselemente die Ausfallsicherheit der Schnittstelle erhöht werden. Als Antriebselemente können Komponenten und/oder Vorrichtungen verstanden werden, die dazu eingerichtet sind, mit der medizinischen Barriere zu interagieren und/oder diese dynamisch zu verformen.

In einigen Ausführungsformen kann die antriebsseitige Kupplungsvorrichtung eine Mehrzahl an Antriebsanordnungen umfassen. Jeder Antriebsanordnung kann ein Freiheitsgrad des medizinischen Instruments zugeordnet sein. **In** anderen Worten kann sich jede Antriebsanordnung unabhängig von anderen Antriebsanordnung bewegen. Der Freiheitsgrad bezieht sich dabei auf die Anzahl der unabhängigen Bewegungen, die das medizinische Instrument ermöglicht. Vorzugsweise sind die Antriebsanordnungen nebeneinander angeordnet. Die Antriebsanordnungen können insbesondere platzsparend positioniert sein, sodass sie nur wenig Bauraum einnehmen.

Gemäß einer Weiterbildung kann die Antriebsanordnung ein bewegbares antriebsseitiges Förderelement umfassen, an und/oder auf welchem die Antriebselemente in Serie angeordnet sind. Eine derartige Ausgestaltung gestattet eine gleichmäßige Kraftverteilung und/oder eine flexible Steuerung der Geschwindigkeit und/oder Förderrichtung der Antriebselemente. Das antriebsseitige Förderelement kann eine antriebsseitige Förderstrecke der Antriebselemente umfassen und/oder definieren und relativ zur Kopplungsfläche und/oder der medizinischen Barriere bewegbar sein. Das antriebsseitige Förderelement kann im Wesentlichen eine Kettenvorrichtung und/oder eine Riemenanordnung, insbesondere in Form einer Förderkette und/oder eines Förderriemens, umfassen, die sich zumindest überwiegend parallel zu der Kopplungsfläche und/oder der medizinischen Barriere erstreckt. Die antriebsseitige Förderstrecke und/oder dessen Verlauf kann je nach Anforderungen und/oder Ausgestaltung der Kopplungsvorrichtung variieren. So kann die antriebsseitige Förderstrecke beispielsweise eine offene oder geschlossene Förderstrecke sein. Beispielsweise kann die die antriebsseitige Förderstrecke und/oder das antriebsseitige Förderelement in Form einer geraden Förderstrecke, also einer linienförmigen Förderstrecke, einer Kurvenförderstrecke, und/oder einer Kreisförderstrecke bzw. eine ringförmigen Förderstrecke ausgebildet sein. Dadurch kann die Förderstrecke und/oder die Schnittstelle besonders kompakt ausgestaltet und/oder der Bauraum effizient genutzt werden. Unter einer offenen Förderstrecke kann eine Förderstrecke verstanden werden, die keinen vollständigen Umlauf der Antriebselemente gestattet. Eine geschlossene Förderstrecke, kann eine Förderstrecke darstellen, auf der die Antriebselemente von einer Ausgangsposition durch Förderung in einer Umlaufrichtung wieder zu dieser Ausgangsposition zurückkehren können. Eine geschlossene Förderstrecke kann zumindest einen vollständigen Umlauf der Antriebselemente entlang der Förderstrecke erlauben. Das Förderelement kann kontinuierlich und/oder diskontinuierlich in wenigstens einer Drehrichtung bewegbar sein. Die Drehrichtung des antriebsseitigen Förderelements kann variierbar sein.

In einer Weiterbildung kann die wenigstens eine Antriebsanordnung wenigstens eine Antriebsquelle umfassen, welche dazu eingerichtet ist, das antriebsseitige Förderelement und/oder wenigstens ein Antriebselement anzutreiben und/oder zu bewegen. Die Antriebsquelle kann dazu eingerichtet sein, zumindest eine Antriebskraft und/oder zumindest ein Antriebsdrehmoment auf das antriebsseitige Förderelement und/oder auf wenigstens ein Antriebselement zu übertragen. Die Antriebsquelle kann insbesondere dazu eingerichtet sein, die Förderrichtung und/oder die Geschwindigkeit, in/mit der sich das Förderelement und/oder die Antriebselemente bewegen sollen, zu steuern. Die Antriebsquelle kann dazu eingerichtet sein, das Förderelement und/oder die Antriebselemente in zwei, insbesondere genau zwei, gegensätzlichen Förderrichtungen relativ zur medizinischen Barriere anzutreiben. Die Antriebsquelle kann eine Antriebswelle sowie wenigstens ein Getriebeelement umfassen. Das wenigstens eine Getriebeelement kann drehmomentübertragbar mit dem antriebsseitigen Förderelement und/oder wenigstens einem Antriebselement koppelbar sein. Das Getriebeelement kann Halte- und/oder Antriebsausnehmungen umfassen, die dazu eingerichtet sind, wenigstens ein Antriebselement zumindest abschnittsweise aufzunehmen und in einer bestimmten Position zu halten und/oder in eine bestimmte Richtung zu bewegen.

Um das antriebsseitige Förderelement bzw. die Antriebselemente in der richtigen Position zu halten und dessen Bewegung zu führen, um eine effiziente Kraftübertragung zu gewährleisten und den Bauraum effizient und/oder platzsparend auszunutzen, kann die antriebsseitige Kopplungsvorrichtung wenigstens ein Umlenkelement umfassen. Das wenigstens eine Umlenkelement kann als eine Umlenkrolle konfiguriert sein.

"In Reihe geschaltet" kann, dass die Antriebselemente derart an und/oder auf dem Förderelement angeordnet und miteinander verbunden sind, dass sie insbesondere in einer Förderrichtung des Förderelements nacheinander, verliersicher und zueinander versetzt angeordnet sind. Vorzugsweise sind die Antriebselemente derart an und/oder auf dem Förderelement angeordnet, dass sich die Antriebselemente bei Bewegung dem Förderelement simultan in einer Förderrichtung bewegen. Die Antriebselemente können derart an und/oder auf dem Förderelement angeordnet sein, dass sie sich zumindest in einem Freiheitsgrad bewegen können. Vorzugsweise sind die Antriebselemente derart an und/oder auf dem Förderelement angeordnet, dass diese zumindest um einen Winkelbetrag um eine Rotationsachse rotierbar sind.

In einigen Ausführungsformen kann wenigstens eines der Antriebselemente zumindest abschnittsweise einen abgerundeten Abschnitt aufweisen. Vorzugsweise ist das wenigstens eine Antriebselement vollständig abgerundet. Abgerundete Abschnitte weisen insbesondere den Vorteil auf, Beschädigungen und/oder Verschleiß innerhalb der Schnittstelle zu verhindern oder zumindest zu minimieren. Insbesondere können Beschädigungen der medizinischen Barriere und damit potenziell verbundene Kontaminationen vorteilhaft vermieden werden.

In einigen Ausführungsformen können alle Antriebselemente zumindest abschnittsweise einen abgerundeten Abschnitt aufweisen. Wenigstens eines der Antriebselemente kann einen kreisförmigen, ringförmigen oder halbkreisförmigen Querschnitt aufweisen. Wenigstens eines der Antriebselemente kann als eine Kugel, eine Halbkugel, ein Zylinder, ein Ring oder ein Halbzylinder ausgebildet sein. Die Antriebselemente können unterschiedlich oder einheitlich ausgebildet sein.

In einigen Ausführungsformen kann die antriebsseitige Kopplungsvorrichtung wenigstens ein Anschlagelement umfassen, das dazu eingerichtet ist, eine Bewegung des Förderelements und/oder der Antriebselemente zuzulassen bzw. ab einem bestimmten Weg oder Betrag zu begrenzen. Auf diese Weise können unerwünschte Belastungen bzw. übermäßige Bewegungen und damit potenzielle Schäden an der Kopplungsvorrichtung bzw. der Schnittstelle verhindert werden. Das wenigstens eine Anschlagelement kann als eine Art Schutzmechanismus betrachtet werden, um die Integrität und Zuverlässigkeit der Schnittstelle zu gewährleisten.

Um Beschädigungen und/oder Verschleiß innerhalb der Schnittstelle zu verhindern oder zumindest zu minimieren, kann die antriebsseitige Kopplungsvorrichtung eine flexible antriebsseitige Membran umfassen. Die flexible antriebsseitige Membran kann im gekoppelten Zustand der Schnittstelle zwischen der Antriebsanordnung und der medizinischen Barriere angeordnet sein. Die antriebsseitige Membran kann die antriebsseitige Kopplungsfläche darstellen. Die flexible antriebsseitige Membran kann durch die Antriebselemente und/oder die instrumentenseitige Kopplungsvorrichtung zumindest in einem Abschnitt wellenförmig bewegbar sein. Die wellenförmige Bewegung der flexiblen antriebsseitigen Membran kann auf die medizinische Barriere und/oder die instrumentenseitige medizinische Kopplungsvorrichtung übertragbar sein. Eine derartige Ausgestaltung senkt das Risiko einer Beschädigung der medizinischen Barriere, da sie verhindert, dass die medizinische Barriere etwa zwischen den Antriebselementen eingeklemmt wird. Aufgrund der Elastizität der Membran, kann diese ausgewölbt und/oder eingedrückt werden und so Bewegungen, insbesondere wellenförmige Bewegungen, bzw. mechanische Energie übertragen und/oder aufnehmen.

Gemäß einer Weiterbildung kann die wenigstens eine instrumentenseitige Kopplungsvorrichtung wenigstens eine zu der wenigstens einen Antriebsanordnung komplementäre Abtriebsanordnung umfassen. Durch die Komplementarität der Abtriebsanordnung kann eine einfache, formschlüssige, und/oder funktionale Kopplung und/oder Montage zwischen der Antriebsanordnung und der Abtriebsanordnung erfolgen. Die wenigstens eine komplementäre Abtriebsanordnung kann dazu eingerichtet sein, Bewegungen der wenigstens einen Antriebsanordnung und/oder Bewegungen der medizinischen Barriere aufzunehmen und/oder wellenförmige Bewegungen auf die wenigstens eine Antriebsanordnung und/oder die medizinische Barriere zu übertragen.

Die wenigstens eine Abtriebsanordnung kann vorzugsweise wenigstens 10 und besonders bevorzugt wenigstens 20 Abtriebselemente umfassen. Eine hohe Anzahl an Abtriebselementen einer Antriebsanordnung kann die Flexibilität und Beweglichkeit der Abtriebsanordnung verbessern. Ferner kann eine hohe Anzahl an Abtriebselementen dazu beitragen, dass Kräfte und/oder Bewegungen gleichmäßiger und/oder präziser zwischen der Antriebsanordnung und der Abtriebsanordnung übertragen werden können. Durch eine redundante Anordnung der Abtriebselemente die Ausfallsicherheit der Schnittstelle erhöht werden. **In** einigen Ausführungsformen kann wenigstens eines der Abtriebselemente zumindest abschnittsweise einen abgerundeten Abschnitt bzw. Querschnitt aufweisen. Wenigstens eines der Abtriebselemente kann einen kreisförmigen, ringförmigen oder halbkreisförmigen Querschnitt aufweisen. Wenigstens eines der Abtriebselemente kann als eine Kugel, eine Halbkugel, ein Zylinder, ein Ring oder ein Halbzylinder ausgebildet sein. Die Abtriebselemente können unterschiedlich oder einheitlich ausgebildet sein.

Vorzugsweise ist das wenigstens eine Abtriebselement vollständig abgerundet. Abgerundete Abschnitte weisen insbesondere den Vorteil auf, Beschädigungen und/oder Verschleiß innerhalb der Schnittstelle zu verhindern oder zumindest zu minimieren. Insbesondere können Beschädigungen der medizinischen Barriere und damit potenziell verbundene Kontaminationen vorteilhaft vermieden werden.

In einer Weiterbildung kann die Abtriebsanordnung ein bewegbares instrumentenseitiges Förderelement aufweisen, an und/oder auf welchem die Abtriebselemente in Serie angeordnet sind. Eine derartige Ausgestaltung gestattet eine gleichmäßige, zuverlässige und/oder effiziente Kraft- und/oder Bewegungsübertragung. Das instrumentenseitige Förderelement kann eine instrumentenseitige Förderstrecke der Abtriebselemente umfassen bzw. definieren und relativ zur instrumentenseitigen Kopplungsfläche bzw. der medizinischen Barriere bewegbar sein. Das instrumentenseitige Förderelement kann im Wesentlichen eine Kettenvorrichtung und/oder eine Riemenanordnung, insbesondere in Form einer Förderkette und/oder eines Förderriemens, umfassen, die sich zumindest überwiegend parallel zu der instrumentenseitigen Kopplungsfläche bzw. der medizinischen Barriere erstreckt. Die instrumentenseitige Förderstrecke bzw. dessen Verlauf kann je nach Anforderungen und/oder Ausgestaltung der instrumentenseitigen Kopplungsvorrichtung variieren. So kann die instrumentenseitige Förderstrecke beispielsweise eine offene oder geschlossene Förderstrecke sein. Beispielsweise kann die instrumentenseitige Förderstrecke bzw. das instrumentenseitige Förderelement in Form einer geraden Förderstrecke, also einer linienförmigen Förderstrecke, einer Kurvenförderstrecke, und/oder einer Kreisförderstrecke bzw. eine ringförmigen Förderstrecke ausgebildet sein. Dadurch kann die Förderstrecke und/oder die Schnittstelle besonders kompakt ausgestaltet und/oder der Bauraum effizient genutzt werden. Das instrumentenseitige Förderelement kann kontinuierlich und/oder diskontinuierlich in wenigstens einer Drehrichtung bewegbar sein. Die Drehrichtung des instrumentenseitigen Förderelements kann variierbar sein.

Die Abtriebsanordnung kann wenigstens ein Getriebeelement umfassen. Das wenigstens eine Getriebeelement kann drehmomentübertragbar mit dem antriebsseitigen Förderelement und/oder wenigstens einem Abtriebselement koppelbar sein. Das wenigstens eine Getriebeelement kann Halte- und/oder Abtriebsausnehmungen umfassen, die dazu eingerichtet sind, wenigstens ein Abtriebselement zumindest abschnittsweise aufzunehmen und in einer bestimmten Position zu halten und/oder in eine bestimmte Richtung zu bewegen.

Um das instrumentenseitige Förderelement bzw. die Abtriebselemente in der richtigen Position zu halten und dessen Bewegung zu führen, um eine effiziente Kraftübertragung zu gewährleisten und den Bauraum effizient bzw. platzsparend auszunutzen, kann die instrumentenseitige Kopplungsvorrichtung wenigstens ein Umlenkelement umfassen. Das wenigstens eine Umlenkelement kann als eine Umlenkrolle konfiguriert sein.

In einigen Ausführungsformen kann die instrumentenseitige Kopplungsvorrichtung wenigstens ein Anschlagelement umfassen, das dazu eingerichtet ist, eine Bewegung des instrumentenseitigen Förderelements und/oder der Abtriebselemente zuzulassen bzw. ab einem bestimmten Weg oder Betrag zu begrenzen. Auf diese Weise können unerwünschte Belastungen bzw. übermäßige Bewegungen und damit potenzielle Schäden an der instrumentenseitigen Kopplungsvorrichtung bzw. der Schnittstelle verhindert werden. Das wenigstens eine Anschlagelement kann als eine Art Schutzmechanismus betrachtet werden, um die Integrität und Zuverlässigkeit der Schnittstelle zu gewährleisten.

In einigen Ausführungsformen können die wenigstens eine Antriebsanordnung und die wenigstens eine komplementäre Abtriebsanordnung identisch ausgebildet sein. Insbesondere kann die Anzahl und/oder Ausgestaltung der Antriebselemente und der Abtriebselemente identisch sein. In alternativen Ausführungsformen können die wenigstens eine Antriebsanordnung und die wenigstens eine komplementäre Abtriebsanordnung unterschiedlich ausgebildet sein.

Um Beschädigungen und/oder Verschleiß innerhalb der Schnittstelle zu verhindern oder zumindest zu minimieren, kann die instrumentenseitige Kopplungsvorrichtung eine flexible instrumentenseitige Membran umfassen, die im gekoppelten Zustand zwischen der Abtriebsanordnung und der medizinischen Barriere angeordnet ist. Die instrumentenseitige Membran kann die instrumentenseitige Kopplungsfläche darstellen. Die flexible instrumentenseitige Membran kann durch die Abtriebselemente und/oder die antriebsseitige Kopplungsvorrichtung zumindest in einem Abschnitt wellenförmig bewegbar sein. Die wellenförmige Bewegung der flexiblen instrumentenseitigen Membran kann auf die medizinische Barriere und/oder die antriebsseitige medizinische Kopplungsvorrichtung übertragbar sein. Eine derartige Ausgestaltung der instrumentenseitigen Kopplungsvorrichtung senkt das Risiko einer Beschädigung der medizinischen Barriere, da sie verhindert, dass die medizinische Barriere etwa zwischen den Antriebselementen eingeklemmt wird.

Aufgrund der Elastizität der Membran, kann diese ausgewölbt und/oder eingedrückt werden und so Bewegungen, insbesondere wellenförmige Bewegungen, bzw. mechanische Energie übertragen und/oder aufnehmen.

In einigen Ausführungsformen kann die antriebsseitige Kopplungsvorrichtung eine Mehrzahl an Antriebsanordnungen umfassen. Zusätzlich oder alternativ kann die instrumentenseitige Kopplungsvorrichtung eine Mehrzahl an Abtriebsanordnungen umfasst. Vorzugsweise entspricht die Anzahl der Antriebsanordnungen der Anzahl der Abtriebsanordnungen.

In einigen Ausführungsformen kann die Schnittstelle der eingangsbezeichneten Art eine Hubvorrichtung umfassen, welche dazu eingerichtet ist, zumindest die Antriebsanordnung funktionsfähig mit der Abtriebsanordnung zu koppeln. Eine funktionsfähige Kopplung kann erfolgen, indem die Antriebsanordnung und/oder die Abtriebsanordnung beispielsweise mittels einer oder mehrerer Hubvorrichtungen, bevorzugt automatisiert in Richtung der jeweils Anordnung verlagert und/oder mit dieser in Eingriff gebracht wird. Unter einer Hubvorrichtung soll hierin insbesondere eine mechanische, hydraulische und/oder pneumatische Einrichtung verstanden werden, die eine vorzugsweise automatisierte Kopplung oder Entkopplung der Antriebsanordnung mit der Abtriebsanordnung ermöglicht. Mittels einer derartigen Hubvorrichtung können präzise Bewegungen ausgeführt werden. Gemäß einigen Ausführungsformen kann die Hubvorrichtung dazu eingerichtet sein, zumindest wenigstens eine Kolbenanordnung relativ zum antriebsseitigen Gehäuse zu verlagern und/oder in einer vorbestimmten Position reversibel zu arretieren. Auf diese Weise kann nicht nur die Kopplung bzw. Entkopplung der Schnittstelle vereinfacht, sondern auch Fehler bzw. Schäden aufgrund fehlerhafter Montage verhindert oder zumindest ein solches Risiko verringert werden.

Alternativ oder zusätzlich kann die Schnittstelle wenigstens eine Federvorrichtung umfassen, welche dazu eingerichtet ist, die Antriebsanordnung und/oder die Abtriebsanordnung bzw. die Antriebselemente und/oder die Abtriebselemente in einer federbelasteten Position vorzuspannen, um eine zuverlässige Bewegungsübertragung zu gewährleisten. Die Federvorrichtung kann insbesondere dazu eingerichtet sein, eine Vorspannkraft zu erzeugen, welche die Antriebselemente in eine bestimmte Position und/oder gegen eine andere Komponente insbesondere Abtriebselemente drückt. Dadurch kann ein ständiger Kontakt zwischen zwei interagierenden Komponenten, insbesondere den Antriebs- und Abtriebselementen, erreicht und die zuverlässige Funktionsweise sichergestellt werden. Die Federvorrichtung kann insbesondere eine metallische und//oder elastomere Schraubenfeder, eine hydraulische Feder und/oder eine pneumatische Feder umfassen.

Gemäß einiger Ausführungsformen können die Antriebs- und Abtriebselemente zumindest abschnittsweise magnetische Eigenschaften aufweisen. Diese Ausführungsform gestatten insbesondere eine zuverlässige Interaktion der Antriebsanordnung mit der Abtriebsanordnung und erlauben eine einfache Montage der Schnittstelle.

Die erfindungsgemäßen Vorrichtungen und Systeme sollen hierbei nicht auf die oben beschriebene Anwendung und Ausführungsform beschränkt sein. Insbesondere können diese zu einer Erfüllung einer hierin beschriebenen Funktionsweise eine von einer hierin genannten Anzahl von einzelnen Elementen, Bauteilen und Einheiten abweichende Anzahl aufweisen. Zudem sollen bei den in dieser Offenbarung angegebenen Wertebereichen auch innerhalb der genannten Grenzen liegende Werte als offenbart und als beliebig einsetzbar gelten.

Im Folgenden wird die vorliegende Erfindung anhand der beigefügten Figuren beispielhaft beschrieben. Die Zeichnungen, die Beschreibung und die Ansprüche enthalten zahlreiche Merkmale in Kombination. Der Fachmann wird die Merkmale zweckmäßigerweise auch einzeln betrachten und im Rahmen der Ansprüche sinnvoll in Kombination verwenden.

Falls von einem bestimmten Objekt mehr als ein Exemplar vorhanden ist, ist ggf. nur eines davon in den Figuren und in der Beschreibung mit einem Bezugszeichen versehen. Die Beschreibung dieses Exemplars kann entsprechend auf die anderen Exemplare von dem Objekt übertragen werden. Sind Objekte insbesondere mittels Zahlenwörtern, wie beispielsweise erstes, zweites, drittes Objekt etc. benannt, dienen diese der Benennung und/oder Zuordnung von Objekten. Demnach können beispielsweise ein erstes Objekt und ein drittes Objekt, jedoch kein zweites Objekt umfasst sein. Allerdings könnten anhand von Zahlenwörtern zusätzlich auch eine Anzahl und/oder eine Reihenfolge von Objekten ableitbar sein.

Es zeigen:
- Fig. 1: ein medizinisches Robotersystem mit einer Schnittstelle zur Verbindung einer Antriebseinheit mit einem medizinischen Instrument,
- Fig. 2: eine perspektivische Darstellung der Schnittstelle mit einer antriebsseitigen Kopplungsvorrichtung und einer instrumentenseitigen Kopplungsvorrichtung in einem aneinander angelegten Zustand,
- Fig. 3: eine seitliche Darstellung der antriebsseitigen Kopplungsvorrichtung und der instrumentenseitigen Kopplungsvorrichtung in einem räumlich voneinander getrennten Zustand,
- Fig. 4: eine perspektivische Darstellung der Schnittstelle bei eingelegter medizinischer Barriere,
- Fig. 5: eine schematische Darstellung der Schnittstelle in dem räumlich voneinander getrennten Zustand,
- Fig. 6: eine schematische Darstellung der Schnittstelle in dem aneinander angelegten Zustand,
- Fig. 7: eine schematische Darstellung einer weiteren Ausführungsform einer Schnittstelle in einem räumlich voneinander getrennten Zustand,
- Fig. 8: eine schematische Darstellung der Schnittstelle gemäß Fig. 7 in einem aneinander angelegten Zustand, und
- Fig. 9: ein Ablaufdiagramm eines Verfahrens zum Verbinden der Kopplungsvorrichtungen unter Zwischenlage der medizinischen Barriere.

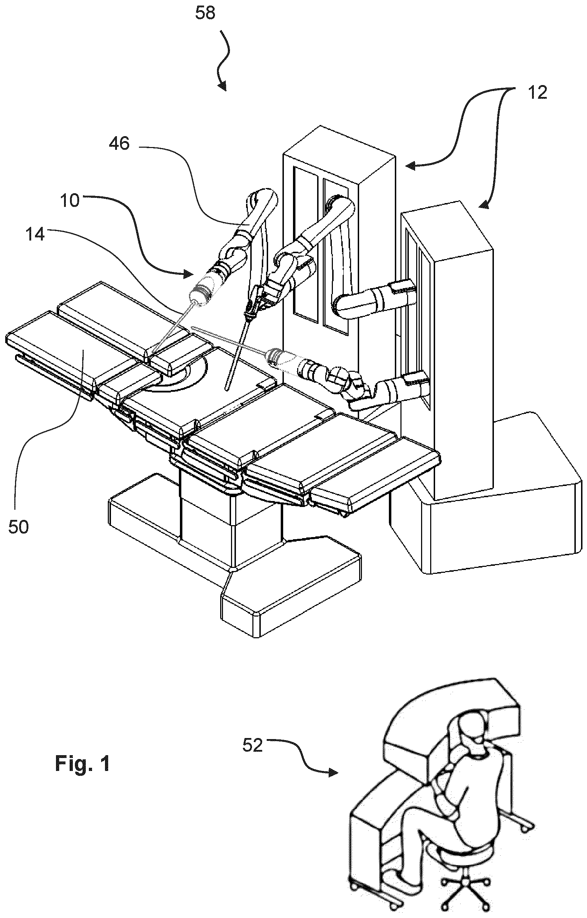

Fig. 1 zeigt ein medizinisches Robotersystem 58. Das Robotersystem 58 weist eine Patientenliege 50 auf. Das Robotersystem weist einen Roboterarm 46 und eine Antriebseinheit 12 auf, wobei der Roboterarm 46 über die Antriebseinheit 12 steuerbar ist. Eine Ansteuerung der Antriebseinheit 12 kann automatisiert, teilautomatisiert und/oder durch medizinisches Personal mittels einer Bedienkonsole 52 erfolgen.

Das Robotersystem weist ein medizinisches Instrument 14 auf, welches an dem Roboterarm 46 angeordnet und durch diesen bewegbar ist. Das medizinische Instrument 14 selbst weist zumindest ein bewegliches Teil auf, welches gegenüber einem weiteren Teil des medizinischen Instruments 14 beweglich ist. Das bewegliche Teil ist durch die Antriebseinheit 12 relativ zu dem weiteren Teil bewegbar. Vorliegend handelt es sich bei dem medizinischen Instrument 14 um eine Zange, wobei auch beliebige andere, dem Fachmann als sinnvoll erscheinende Instrumente denkbar wären. Der Übersichtlichkeit halber sind nicht alle Komponenten des Robotersystems mit Bezugszeichen versehen.

Um sterile Arbeitsbedingungen zu schaffen, weist das Robotersystem eine durchgängige medizinische Barriere 24 auf, welche einen sterilen Bereich um die Patientenliege 50 von der potenziell unsterilen Antriebseinheit 12 und dem Roboterarm 46 trennt. Eine Antriebskraft und/oder ein Antriebsdrehmoment für das bewegliche Teil des medizinische Instrument 14 wird mittels einer Schnittstelle 10 des Robotersystems übertragen. Die Schnittstelle 10 ist in einem Einsatzzustand im Bereich der medizinischen Barriere 24 angeordnet. Die Schnittstelle 10 ist in dem Einsatzzustand durch den Roboterarm 46 gehalten und geführt.

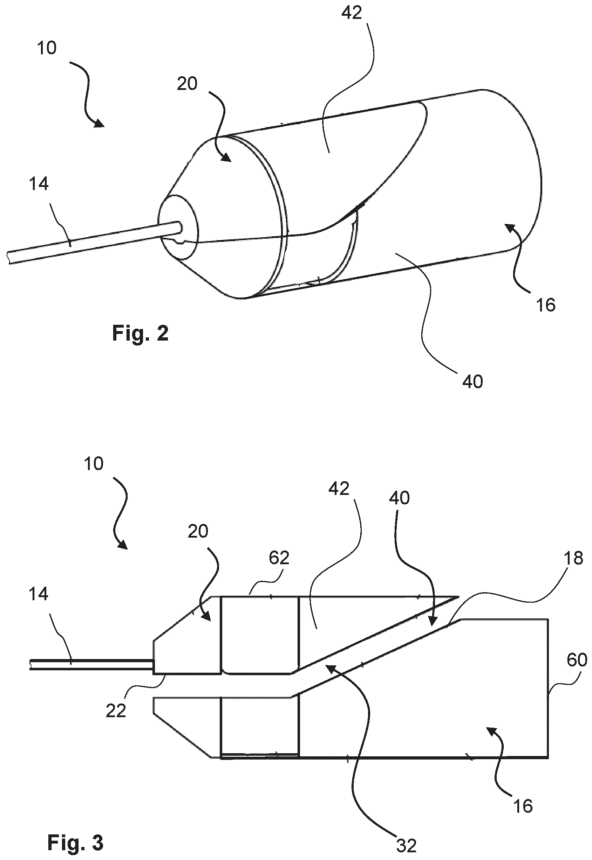

Die Schnittstelle 10 weist eine antriebsseitiges Kopplungsvorrichtung 16 und eine instrumentenseitige Kopplungsvorrichtung 20 auf, welche in Fig. 2 in einer perspektivischen Darstellung in einem aneinander angelegten Zustand gezeigt sind. Fig. 3 zeigt die Kopplungsvorrichtungen 16, 20 in einem voneinander räumlich getrennten Zustand. Die Kopplungsvorrichtungen 16, 20 weisen jeweils ein Gehäuse 60, 62 auf, die verliersicher miteinander verbindbar sind. Die Verbindung der beiden Gehäuse 60, 62 ist lösbar.

Die antriebsseitige Kupplungsvorrichtung 16 weist eine antriebsseitige Kopplungsfläche 18 auf. Die instrumentenseitige Kopplungsvorrichtung 20 umfasst eine instrumentenseitige Kopplungsfläche 22. Die Kopplungsflächen 18, 22 sind zueinander komplementär ausgebildet und erlauben ein formschlüssiges Zusammenfügen der Verbindungselemente 16, 20, wie in Fig. 2 dargestellt.

Die Kopplungsvorrichtungen 16, 20 und/oder deren Gehäuse 60, 62 bilden zusammengesetzt einen zumindest teilweise kreiszylindrischen und zumindest teilweise kegelstumpfförmigen Körper (vgl. Fig. 2 und 3).

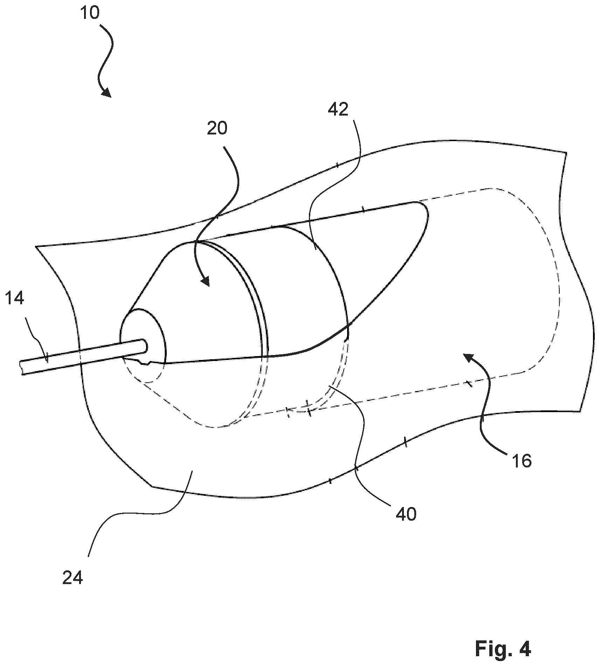

Die Kopplungsflächen 18, 22 sind dazu eingerichtet, die medizinische Barriere 24 einzuklemmen (vgl. Fig. 4). Die Kopplungsvorrichtungen 16, 20 sind über ihre jeweiligen Kopplungsflächen 18, 22 unter Zwischenschaltung der medizinischen Barriere 24 miteinander koppelbar. Bei der medizinischen Barriere 24 handelt es sich um eine durchgängige Kunststofffolie, welche insbesondere frei von Öffnungen ist. In dem Einsatzzustand sind die beiden Kopplungsvorrichtungen 16, 20 mittels der medizinischen Barriere 24 voneinander getrennt. Dabei befindet sich die instrumentenseitige Kopplungsvorrichtung 20 zusammen mit dem medizinischen Instrument 14 in dem Einsatzzustand auf einer sterilen Seite der medizinischen Barriere 24. Die antriebsseitige Kopplungsvorrichtung 16 wird in dem in Figur 4 gezeigten Einsatzzustand von der medizinischen Barriere 24 weitestgehend verdeckt, was durch die gestrichelten Linien angedeutet ist.

Die instrumentenseitige Kopplungsvorrichtung 20 und/oder deren Gehäuse 62 kann beispielsweise über eine hierin nicht dargestellte Klemmschelle und/oder mittels einer sonstigen vorteilhaften, vorzugsweise reversiblen, Befestigungsvorrichtung verliersicher und funktional mit der antriebsseitigen Kopplungsvorrichtung 16 und/oder deren Gehäuse 60 zusammengehalten werden.

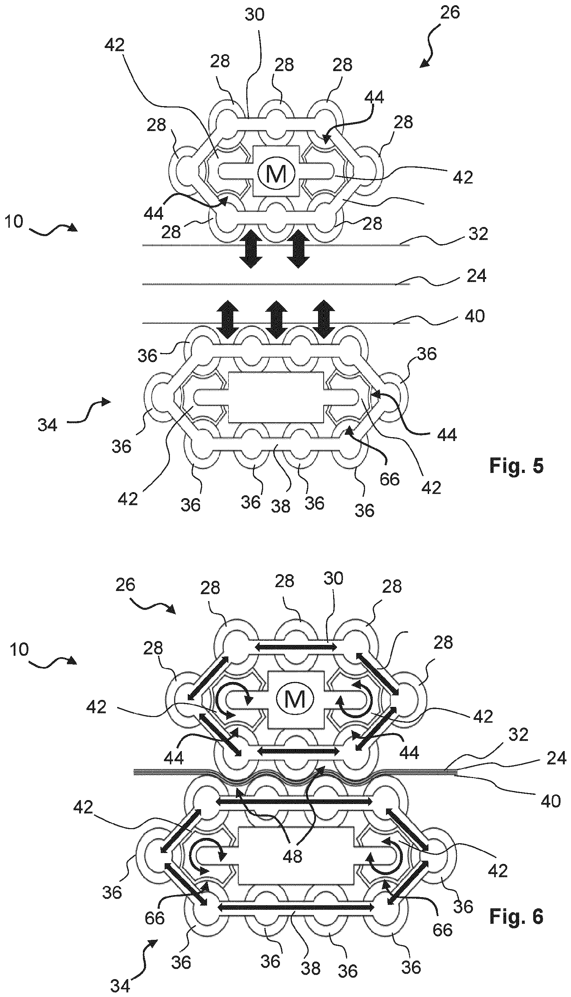

Eine Übertragung einer Antriebskraft und/oder eines Antriebsdrehmoments durch die durchgängige medizinische Barriere 24 erfolgt mechanisch über wenigstens eine Antriebsanordnung 26, die bewegungsübertragbar mit wenigstens einer komplementären Abtriebsanordnung 34 gekoppelt ist. In Fig. 5 ist eine schematische Darstellung der Schnittstelle 10 im geöffneten, also nicht gekoppelten Zustand gezeigt. Der Übersichtlichkeit halber wurde das antriebsseitige Gehäuse 60 in dieser Darstellung ausgeblendet. Im oberen Abschnitt der Fig. 5 ist die Antriebsanordnung 26 gezeigt. Diese umfasst ein antriebsseitiges Förderelement 30, dass in der hierin gezeigten Ausführungsform ringförmig konfiguriert ist. Entlang des antriebsseitigen Förderelements 30 sind in regelmäßigen Abständen Antriebselemente 28 angeordnet. Die Antriebselemente 28 weisen in dieser Ausführungsform einen kreisförmigen Querschnitt auf. Ferner umfasst die Antriebsanordnung 26 eine Antriebsquelle M mit zwei Getriebeelementen 42. Jedes der beiden Getriebeelemente 42 weist vier Antriebsausnehmungen 44 auf. In jeweils drei der vier Antriebsausnehmungen 44 ist abschnittsweise eines von insgesamt acht Antriebselementen 28 aufgenommen. Die Antriebsausnehmungen 44 sind derart konfiguriert, dass die darin aufgenommenen Antriebselemente 28, im Falle einer Drehbewegung eines der Getriebeelemente 42, entlang einer Förderrichtung des antriebsseitigen Förderelements 30 bewegt werden und die Antriebsausnehmungen 44 nach einer 180° Drehung eines jeweiligen Getriebeelements 42 verlassen können. An dieser Stelle sei darauf hingewiesen, dass aus Gründen der Übersichtlichkeit in der hierin und auch in den nachfolgend beschriebenen Figuren nicht alle Bauteile mit Bezugszeichen versehen sind.

Im unteren Abschnitt der Fig. 5 ist die Abtriebsanordnung 34 dargestellt. Der Grundaufbau der Abtriebsanordnung 34 gleicht im Wesentlichen dem Aufbau der voranstehend beschriebenen Antriebsanordnung 26. Der Hauptunterschied liegt in der Anzahl der an einem instrumentenseitigen Förderelement 38 angeordneten Abtriebselemente 36. In der hierin gezeigten Ausführungsform weist die Abtriebsanordnung 34 insgesamt zehn Abtriebselemente 36 auf. Ein weiterer Unterschied in der hierin gezeigten Ausführungsform besteht darin, dass die Abtriebsanordnung 34 im Vergleich zur Antriebsanordnung 26 keine Antriebsquelle M aufweist.

Die Antriebsanordnung 26 und die Abtriebsanordnung 34 werden an jeweils sich gegenüberliegenden Seiten von einer flexiblen antriebsseitigen und/oder instrumentenseitige Membran 32, 40 bedeckt. Die Membranen 32, 40 sind dazu eingerichtet, zumindest abschnittsweise die Antriebselemente 28 und/oder die Abtriebselemente 36 zu bedecken. Eine derartige Ausgestaltung senkt insbesondere das Risiko einer Beschädigung einer medizinischen Barriere 20, da sie verhindert, dass die medizinische Barriere 20 etwa zwischen den Antriebselementen 28 und/oder Abtriebselementen 36 und den Getriebeelemente 42 eingeklemmt und möglicherweise beschädigt wird. Aufgrund der Elastizität der Membranen 32, 40 können diese mechanisch ausgewölbt und/oder eingedrückt werden und so Bewegungen bzw. mechanische Energie übertragen und/oder aufnehmen.

Die Pfeile in Fig. 5 sollen andeuten, dass die Antriebsanordnung 26 und/oder die Abtriebsanordnung 34 dazu eingerichtet sein können senkrecht zu den Membranen 32, 40 und/oder der medizinischen Barriere 24 bewegbar zu sein. Eine derartige Ausgestaltung gestattet es die Antriebsanordnung 26 funktionsfähig mit der Abtriebsanordnung 34 zu koppeln. Eine funktionsfähige Kopplung kann erfolgen, indem die Antriebsanordnung 26 und/oder die Abtriebsanordnung 34 beispielsweise mittels einer oder mehrerer in dieser Figur nicht gezeigten Hubvorrichtungen und/oder Federvorrichtungen, bevorzugt automatisiert in Richtung der jeweils anderen Anordnung verlagert und/oder mit dieser in Eingriff gebracht wird. Zusätzlich oder alternativ können sich die Antriebselemente 28 und Abtriebselemente 36 auch aufgrund von magnetischen Eigenschaften aufeinander zu bewegen.

Fig. 6 zeigt eine schematische Darstellung der Schnittstelle 10 im gekoppelten Zustand. **In** diesem Zustand kontaktieren die antriebsseitige Kopplungsfläche 18 und/oder die antriebsseitige Membran 32 sowie die instrumentenseitige Kopplungsfläche 22 und/oder die instrumentenseitige Membran 40 die medizinische Barriere 24. Ferner sind die Antriebsanordnung 26 und die Abtriebsanordnung 34 derart positioniert, dass einige der Antriebselemente 28 zumindest abschnittsweise in zwischen den Abtriebselementen 36 ausgebildete Zwischenräume 48 und einige der Abtriebselemente 36 zumindest abschnittsweise in zwischen den Antriebselementen 28 ausgebildeten Zwischenräume 48 hineinragen und/oder sich in den Zwischenräumen 48 erstrecken. Dadurch werden die antriebsseitige Membran 32, die medizinische Barriere 24 sowie die instrumentenseitige Membran verformt. **In** der Fig. 6 ist eine Momentaufnahme und/oder ein Ruhezustand der Schnittstelle 10 gezeigt. Die antriebsseitige Membran 32, die medizinische Barriere 24 sowie die instrumentenseitige Membran 40 weisen im hier gezeigten Querschnitt einen sinusförmigen Wellenverlauf auf.

Die in Fig. 6 eingezeichneten Pfeile sollen Freiheitsgrade der Getriebeelemente 42 und der Förderelemente 30, 38 andeuten. Da die Förderelemente 30, 38 gemäß der hierein gezeigten Ausführungsform ringförmig konfiguriert sind, bzw. die Förderstrecke geschlossen ist, können die Förderelemente 30, 38 kontinuierlich in einer Richtung bewegt werden. In anderen Worten können die Antriebselemente 28 und die Abtriebselemente 36 zumindest einen vollständigen Umlauf entlang einer jeweiligen Förderstrecke durchlaufen. Die Antriebselemente 28 und die Abtriebselemente 36 können um eine Rotationsachse, die sich jeweils durch einen Mittelpunkt der Antriebs- und/oder Abtriebselemente in die Zeichenebene hineinerstreckt, rotierbar sein. Der Übersichtlichkeit halber ist dieser Freiheitsgrad nicht eingezeichnet.

Sobald die Antriebsanordnung 26 und/oder das antriebsseitige Förderelement 30 entgegen des Uhrzeigersinns bewegt wird, drücken die Abtriebselemente 36 die antriebsseitige Membran 32, die medizinische Barriere 24 sowie die instrumentenseitige Membran 40 gegen die Abtriebselemente 36, sodass die Abtriebselemente 36 in die Richtung gedrückt werden, die der Förderrichtung der Antriebselemente 28, also entgegen dem Uhrzeigersinn entspricht. Dadurch, dass sich sowohl die Antriebselemente 28 als auch die Abtriebselemente 36 relativ zu den Membranen 32, 40 und der medizinischen Barriere 24 bewegen, "wandert" bzw. breitet sich auch die auf den Membranen 32, 40 und der medizinischen Barriere 24 erzeugte Welle in Förderrichtung aus. Die voranstehenden Ausführungen sind lediglich beispielhaft zu verstehen. Es versteht sich von selbst, dass die gleichen Ausführungen auch gelten, wenn sich die Förderelemente 30, 38 bzw. Antriebselemente 28 und Abtriebselemente 36 in entgegengesetzter Richtung, also im Uhrzeigersinn, bewegen. Ferner sei an dieser Stelle sei darauf hingewiesen, dass die Antriebselemente 28 und die Abtriebselemente 36 jederzeit in einer bestimmten Position gehalten werden und/oder jederzeit ein Richtungswechsel der Förderrichtung erfolgen kann. In anderen Betriebszuständen können auch Bewegungen von den Abtriebselementen 36 auf die Antriebselemente 28 übertragen werden.

Die vorangehend beschriebene Kombination aus Antriebsanordnung 26 und Abtriebsanordnung 34, kann einen Freiheitsgrad des medizinischen Instruments 14 bilden. Die vorangehen beschriebene Kombination aus Antriebsanordnung 26 und Abtriebsanordnung 34, kann beispielhaft für einen jeweiligen Freiheitsgrad des medizinischen Instruments 14 verstanden werden. Das medizinische Instrument 14 kann mehrere Freiheitsgrade aufweisen, wobei es für jeden Freiheitsgrad eine vorangehend beschriebene Kombination aus Antriebsanordnung 26 und Abtriebsanordnung 34 geben kann,

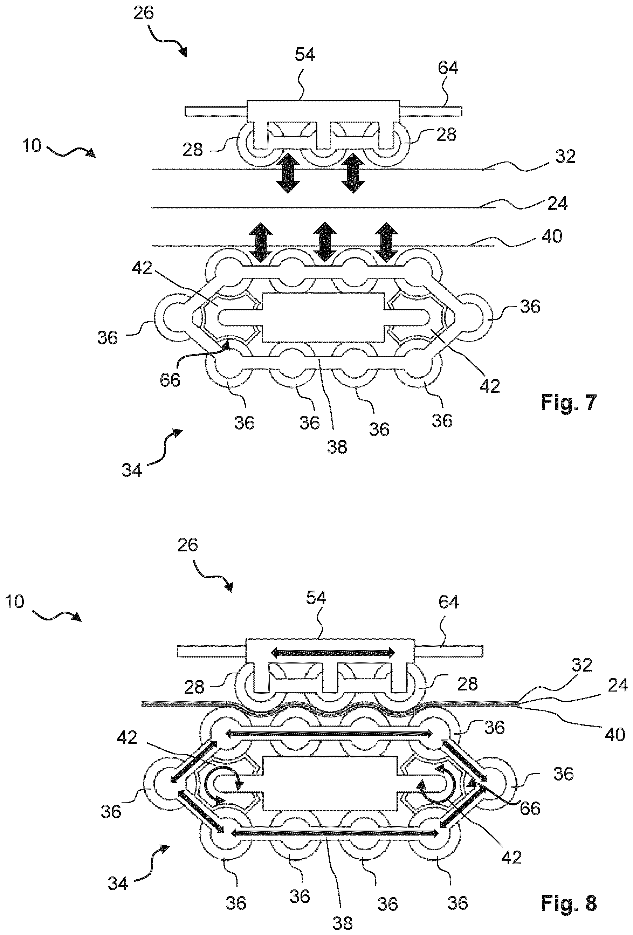

Eine alternative Ausführungsform einer Schnittstelle 10 ist in den Figuren 7 und 8 gezeigt. Der Wesentliche Unterschied zu der vorangehend gezeigten Ausführungsform liegt in der Ausgestaltung einer Antriebsanordnung 26. Gemäß dieser alternativen Ausführungsform ist die Antriebsanordnung 26 und/oder eine antriebsseitige Förderstrecke nicht wie in der in den Figuren 5 und 6 gezeigten Ausführungsform geschlossen, sondern offen ausgebildet. Ein antriebsseitige Förderelement 30 ist als eine Art Schlitten 54 konfiguriert, der mittels einer Seilvorrichtung 64 oder einer Kette der Antriebsanordnung 26 parallel zu einer antriebsseitigen Membran 32 und/oder einer medizinischen Barriere 24 verlagert werden kann. Der Schlitten 54 kann sich nicht kontinuierlich in einer Förderrichtung bewegen. Der Schlitten 54 muss nach einer maximalen Verlagerung wieder in eine Ausgangsposition oder zumindest in eine entgegengesetzte Richtung bewegt oder zurückgesetzt werden, ohne dabei einen gesamten Umlauf entlang der Förderstrecke zu durchlaufen.

Die in den Figuren 7 und 8 gezeigte Kombination aus Antriebsanordnung 26 und Abtriebsanordnung 34, kann einen Freiheitsgrad des medizinischen Instruments 14 bilden. Die in den Figuren 7 und 8 gezeigte Kombination aus Antriebsanordnung 26 und Abtriebsanordnung 34, kann beispielhaft für einen Freiheitsgrad des medizinischen Instruments 14 verstanden werden. Das medizinische Instrument 14 kann mehrere Freiheitsgrade aufweisen, wobei es für jeden Freiheitsgrad eine vorangehend beschriebene Kombination aus Antriebsanordnung 26 und Abtriebsanordnung 34 geben kann, Die Freiheitsgrade des medizinischen Instruments 14 können durch unterschiedliche Kombinationen aus Antriebsanordnung 26 und Abtriebsanordnung 34 ausgebildet sein. Beispielsweise kann ein Freiheitsgrad des medizinischen Instruments von der in den Figuren 5 und 6 gezeigten Kombination aus Antriebsanordnung 26 und Abtriebsanordnung 34 gebildet werden und ein anderer Freiheitsgrad des medizinischen Instruments 14 von der in den Figuren 7 und 8 gezeigten Kombination aus Antriebsanordnung 26 und Abtriebsanordnung 34.

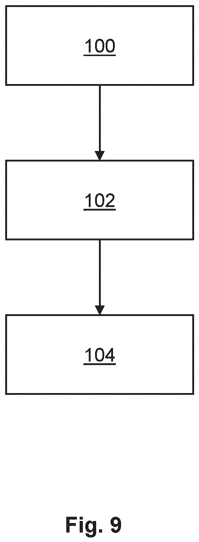

Fig. 9 zeigt ein Ablaufdiagramm eines Verfahrens zum Verbinden der Kopplungsvorrichtungen 16, 20 unter Zwischenlage der medizinischen Barriere 24. In einem Schritt 100 wird die erste Kopplungsfläche 18 und das Gehäuse 60 der antriebsseitigen Verbindungselements 16 zumindest abschnittsweise mit der medizinischen Barriere 24 bedeckt. In einem Schritt 102 wird die instrumentenseitige Kopplungsvorrichtung 20 über seine instrumentenseitige Kopplungsfläche 22 unter Zwischenlage der medizinischen Barriere 24 mit der ersten Kopplungsfläche 18 verbunden. In einem Schritt 104 werden schließlich die Antriebselemente 28 der Antriebsanordnung 26 bewegungsübertragbar mit den Abtriebselementen 36 der Abtriebsanordnung 34 gekoppelt.

### Bezugszeichenliste

- 10: Schnittstelle
- 12: Antriebseinheit
- 14: medizinisches Instrument
- 16: antriebsseitige Kopplungsvorrichtung
- 18: antriebsseitige Kopplungsfläche
- 20: instrumentenseitige Kopplungsvorrichtung
- 22: instrumentenseitige Kopplungsfläche
- 24: medizinische Barriere
- 26: Antriebsanordnung
- 28: Antriebselement
- 30: antriebsseitiges Förderelement
- 32: antriebsseitige Membran
- 34: Abtriebsanordnung
- 36: Abtriebselement
- 38: instrumentenseitiges Förderelement
- 40: instrumentenseitige Membran
- 42: Getriebeelement
- 44: Antriebsausnehmung
- 46: Roboterarm
- 48: Zwischenraum
- 50: Patientenliege
- 52: Bedienkonsole
- 54: Schlitten
- 58: Medizinisches System
- 60: antriebsseitiges Gehäuse
- 62: instrumentenseitiges Gehäuse
- 64: Seilvorrichtung
- 66: Abtriebsausnehmung
- 100: Schritt
- 102: Schritt
- 104: Schritt
- M: Antriebsquelle

## Patentansprüche

1. Schnittstelle (10) zur Verbindung einer Antriebseinheit (12) mit einem medizinischen Instrument (14), umfassend:
- wenigstens eine antriebsseitige Kopplungsvorrichtung (16), und
- wenigstens eine zu der antriebsseitigen Kopplungsvorrichtung (16) komplementäre instrumentenseitige Kopplungsvorrichtung (20),
wobei die antriebsseitige und die instrumentenseitige Kopplungsvorrichtung (16, 20) unter Zwischenschaltung einer durchgängigen medizinischen Barriere (24) mechanisch miteinander koppelbar sind, und
wobei zumindest die antriebsseitige Kopplungsvorrichtung (16) wenigstens eine Antriebsanordnung (26) umfasst, welche dazu eingerichtet ist, zumindest in einem Abschnitt der medizinischen Barriere (24) eine wellenförmige Bewegung der medizinischen Barriere (24) zu erzeugen.

2. Schnittstelle (10) nach Anspruch 1,
wobei die wellenförmige Bewegung entlang wenigstens einer Ausbreitungsrichtung erfolgt.

3. Schnittstelle (10) nach einem der vorherigen Ansprüche,
wobei die Antriebsanordnung (26) wenigstens zwei, insbesondere wenigstens 5, vorzugsweise wenigstens 10 und besonders bevorzugt wenigstens 20 Antriebselemente (28) zur Erzeugung der wellenförmigen Bewegung umfasst.

4. Schnittstelle (10) nach Anspruch 3,
wobei die Antriebsanordnung (26) ein bewegbares antriebsseitiges Förderelement (30) umfasst, an und/oder auf welchem die Antriebselemente (28) in Serie angeordnet sind.

5. Schnittstelle (10) nach einem der Ansprüche 3 oder 4,
wobei wenigstens eines der Antriebselemente (28) zumindest abschnittsweise einen abgerundeten Querschnitt aufweist.

6. Schnittstelle (10) nach einem der Ansprüche 3 bis 5,
wobei das antriebsseitige Förderelement (30) ringförmig oder linienförmig ausgebildet ist.

7. Schnittstelle (10) nach einem der vorherigen Ansprüche,
wobei die antriebsseitige Kopplungsvorrichtung (16) eine flexible antriebsseitige Membran (32) umfasst, die im gekoppelten Zustand zwischen der Antriebsanordnung (26) und der medizinischen Barriere (24) angeordnet ist,
wobei die flexible antriebsseitige Membran (32) durch die Antriebselemente (28) und/oder die instrumentenseitige Kopplungsvorrichtung (20) zumindest in einem Abschnitt wellenförmig bewegbar ist, und wobei die wellenförmige Bewegung der flexiblen antriebsseitigen Membran (32) auf die medizinische Barriere (24) und/oder die instrumentenseitige Kopplungsvorrichtung (20) übertragbar ist.

8. Schnittstelle (10) nach einem der vorherigen Ansprüche,
wobei die wenigstens eine instrumentenseitige Kopplungsvorrichtung (20) wenigstens eine zu der wenigstens einen Antriebsanordnung (26) komplementäre Abtriebsanordnung (34) umfasst, die dazu eingerichtet ist, Bewegungen der wenigstens einen Antriebsanordnung (26) und/oder Bewegungen der medizinischen Barriere (24) aufzunehmen und/oder wellenförmige Bewegungen auf die wenigstens eine Antriebsanordnung (26) und/oder die medizinische Barriere (24) zu übertragen.

9. Schnittstelle (10) nach Anspruch 8,
wobei die Abtriebsanordnung (34) wenigstens zwei, insbesondere wenigstens 5, vorzugsweise wenigstens 10 und besonders bevorzugt wenigstens 20 Abtriebselemente (36) umfasst.

10. Schnittstelle (10) nach Anspruch 9,
wobei die Abtriebsanordnung (34) ein bewegbares abtriebsseitiges Förderelement (38) aufweist, an und/oder auf welchem die Abtriebselemente (36) in Serie angeordnet sind.

11. Schnittstelle (10) nach einem der Ansprüche 9 oder 10,
wobei wenigstens eines der Abtriebselemente (36) zumindest abschnittsweise einen abgerundeten Querschnitt aufweist.

12. Schnittstelle (10) nach Anspruch 10 oder 11,
wobei das abtriebsseitige Förderelement (38) ringförmig oder linienförmig ausgebildet ist.

13. Schnittstelle (10) nach einem der vorherigen Ansprüche 8 bis 12,
wobei die instrumentenseitige Kopplungsvorrichtung (20) eine flexible abtriebsseitige Membran (40) umfasst, die im gekoppelten Zustand zwischen der Abtriebsanordnung (34) und der medizinischen Barriere (24) angeordnet ist,
wobei die flexible abtriebsseitige Membran (40) durch die Abtriebselemente (36) und/oder die instrumentenseitige Kopplungsvorrichtung (20) zumindest in einem Abschnitt wellenförmig bewegbar ist, und wobei die wellenförmige Bewegung der flexiblen abtriebsseitigen Membran (40) auf die medizinische Barriere (24) und/oder die antriebsseitige Kopplungsvorrichtung (16) übertragbar ist.

14. Schnittstelle (10) nach einem der vorherigen Ansprüche 8 bis 13,
wobei die antriebsseitige Kopplungsvorrichtung (16) eine Mehrzahl an Antriebsanordnungen (26) umfasst, und/oder wobei die instrumentenseitige Kopplungsvorrichtung (20) eine Mehrzahl an Abtriebsanordnungen (34) umfasst.

15. Medizinisches Robotersystem (58) umfassend eine Schnittstelle (10) nach einem der vorherigen Ansprüche.
